# EUROPEAN PATENT SPECIFICATION

(11) **EP 0 565 134 B1**
(45) Date of publication and mention of the grant of the patent: **20.03.1996**
(21) Application number: 93108221.8
(22) Date of filing: 13.04.1988
(51) Int. Cl.: A61K 33/20, A61K 7/20

(54) **Disinfecting compositions and methods therefor**
Desinfizierende Zusammensetzung und Verfahren zu ihrer Herstellung
Compositions désinfectantes et procédé pour leurs obtentions

(30) Priority: 14.04.1987 US 38016
(43) Date of publication of application: 13.10.1993
(62) Divisional of application: 88105893.7
(73) Proprietor: ALCIDE CORPORATION, Redmond, WA 98052 (US)
(72) Inventor: Kross, Robert D., Bellmore, New York 11710 (US); Zamojcin, Carol A., Floral Park, New York 11001 (US)
(74) Representative: Grünecker, Kinkeldey, Stockmair & Schwanhäusser Anwaltssozietät

(56) References cited:
- AU-A- 545 171
- FR-A- 2 380 774

## Description

### Technical Field

This invention relates generally to compositions for disinfecting substrates as well as to processes for preparing and using such compositions. Further, this invention relates to disinfecting compositions useful as oral hygiene compositions such as a mouthwash, toothpaste, lozenge, chewing gum, or the like.

### Background of the Invention

The term "disinfectant" is used in this specification to broadly include any substance or composition that disinfects, sanitizes, deodorizes, sterilizes, or kills germs.

The use of chlorine compounds in various types of disinfectant compositions is well known. Chlorine compounds suggested for use in this regard include, for example, sodium hypochlorite, used in World War I was a wound irrigant, and chlorinated phenols, such as m-chlorophenol. These compounds have increased bactericidal activity and reduced toxicity, in some instances, when compared to non-chlorinated phenols. Thus, m-chlorophenol has a phenol coefficient of 5.8 (S. aureus) to 7.4 (B. typhosus). Other chlorine compounds having some form of disinfectant utility include, for example, chlorine gas itself, chlorine dioxide, chloramine T, calcium hypochlorite (a standard swimming pool disinfectant), chloropicrin (a larvicide), chloroform (a fumigant), chlorodane (an insecticide), and chloromycetin (an antibiotic).

Chlorine dioxide in particular has been found to be an especially effective disinfectant. This compound is quite versatile and has long been used as a bleaching agent, such as in the oxidizing of the natural colorant present in cotton, wood-pulp and other cellulosic fibrous material. In such uses, chlorine dioxide, though performing an oxidizing function, is nevertheless noninjurious with respect to the fibrous material.

Particularly useful disinfecting compositions which employ chlorine-containing compounds are disclosed in U.S. Patent Nos. 4,330,531 and 4,585,482. These compositions comprise chlorite salts, such as sodium chlorite, in combination with weak organic acids, such as lactic acid, to provide particularly effective disinfectant compositions. These compositions are useful in topically treating skin diseases such as acne and in toothpastes.

One particularly prevalent disease is mastitis in dairy herds. Mastitis is a highly infectious disease which affects the bovine udder. The losses in dairy production resulting from this disease are staggering. For example, in the United States alone, these losses are estimated to be in the hundreds of millions of dollars. Mastitis not only reduces the production of higher yielding animals, but also shortens their productive life.

It is well known that mastitis is transmitted from animal to animal. It has also been established that the only route of transmission of the disease is through the teat orifice. Conditions which are primarily responsible for the high incidence of mastitis include poor udder hygiene and physical damage to the teats. Dairymen and veterinarians have long sought a conditioning and protective composition which is economical and also provides facility of use. A composition which would improve the normal condition of the udder and teats and would also aid in preventing or effectively reducing the incidence of mastitis would serve to substantially increase both the production and productive life of a dairy herd.

Infectious mastitis is caused by microorganisms. Prior art treatments, such as the use of sulfanilimide, have been only partially effective in controlling the disease because such treatments are useful against only one type of microorganism which causes mastitis but are not useful against other types. Since the infection is usually of a mixed character, it follows that the effectiveness of drugs such as sulfanilimide is limited for all practical purposes.

It has also been suggested to use penicillin for the treatment of mastitis. The use of this compound is not desirable, however, in view of the high cost and extraordinary conditions necessary for the preservation and use of penicillin.

It has been proposed in U.S. Patent No. 3,222,252 to treat mastitis with a preparation which comprises a blend of edible, semidrying oils and drying oils together with a fatty acid ester, skin emollient, film-forming agent. It is said that the presence of a drying oil in the preparation is essential to provide the desired film-forming property. But for the most part, these film-forming-based treatments of mastitis have been unsuccessful. The use of iodine and peroxide in conjunction with latex emulsion films has led to other problems, including milk contamination and skin irritation.

While chlorine-liberating compounds have germicidal and deodorant properties, their characteristic taste and odor make them unpleasant for use in oral hygiene compositions such as mouthwashes. Other oral hygiene compositions, some commercially available, while pleasant tasting, are ineffective to reduce dental plaque as well as control gingivitis and periodontitis.

The search has continued for new and improved oral hygiene compositions which are both pleasant in tasting and effective in reducing dental plaque as well as control gingivitis and periodontitis. This invention was made as a result of that search.

The use of compositions comprising metal chlorite and a weak organic acid, such as lactic acid, is disclosed in U.S. Patent No. 4,330,531. A chlorine dioxide releasing compound offers to substantially alleviate the problems associated with the prevention of mastitis. For this application, the use of a gelling agent to generate a viscous topical gel is essential. The viscosity is needed for proper adhesion to the skin surface for an extended length of time.

Past attempts to create a useful viscous topical gel containing chlorine dioxide releasing compounds have been less than completely successful. Of the gelling agents tried, none combined the properties needed for substantial effectiveness. Carbohydrate-based gels lose their viscosity at higher pH's as the alkaline chlorites break down the long polymer chains. Inorganic thickeners, such as bentonite clays, participate in the reaction between the acid and chlorite, and tend to form stringy, unsuitable gels.

The search has continued for a viscous topical gel composition capable of embodying alkaline chlorites for the treatment and prevention of skin disease, infection and irritation on humans and other animals. This invention was made as a result of that search.

### Objects and Summary of the Invention

Accordingly, it is a general object of the present invention to avoid or substantially alleviate the above-identified problems of the prior art.

A further object of the present invention is to provide oral hygiene compositions which are pleasant tasting yet effective to reduce dental plaque and control gingivitis and periodontitis.

An additional object of this invention is to provide a method for producing these oral hygiene compositions.

Other objects and advantages of the present invention will become apparent from the following summary of the invention and description of its preferred embodiments.

The present invention provides, in one aspect, a disinfectant oral hygiene composition for reducing dental plaque and controlling gingivitis and periodontitis. This composition comprises: (a) a flavoring agent acceptable for oral hygiene compositions, (b) an aqueous solution containing a suitable amount of a protic acid, and (c) an amount of a metal chlorite such that the chlorite ion concentration in the form of chlorous acid is no more than about 15 percent by weight of the total amount of chlorite ion concentration. The composition contains substantially no lactic acid.

In another aspect, the present invention provides a process for producing these disinfectant oral hygiene compositions. This process comprises admixing (a) a flavoring agent acceptable for oral hygiene compositions, (b) from about 0.03 to about 0.3 percent by weight of a chlorine dioxide liberating compound based upon the total weight of the composition, and (c) a suitable amount of an organic acid having a pK of from about 2.8 to about 4.2. The composition contains substantially no lactic acid.

A process for reducing dental plaque in the mouth comprises treating the mouth with a composition which comprises: (a) a flavoring agent acceptable for oral hygiene compositions, (b) from about 0.03 to about 0.3 percent by weight of a chlorine dioxide liberating compound based upon the total weight of the composition, and (c) a suitable amount of an organic acid having a pK of from about 2.8 to about 4.2. The composition contains substantially no lactic acid.

The disinfecting compositions of this invention disinfect by means of a chlorous acid generating composition. The composition comprises a flavoring agent, suitable amount of a carboxylic acid, and a suitable amount of a metal chlorite. The chlorite ion concentration in the form of chlorous acid is no more than about 15 percent by weight of the total amount of chlorite ion concentration.

The oral hygiene formulations of this invention contain low concentrations of chlorous acid generating compounds which are extremely effective as oral disinfectants. These compositions provide effective mouthwashes, toothpastes and other oral disinfecting compositions without the strong characteristic unpleasant taste of chlorine.

The oral hygiene compositions of this invention comprise a flavoring agent suitable for use in an oral hygiene composition, a suitable amount of a carboxylic acid, and a suitable amount of a metal chlorite. The concentrations of chlorite and acid are relatively low and are such that the amount of chlorite in the form of chlorous acid is no more than about 15 percent by weight of the total chlorite ion concentration in solution. Typically the amount of the chlorite in the form of chlorous acid is nor more than about 10 percent by weight of the total chlorite ion concentration in solution.

The percent by weight of chlorite and chlorous acid may be calculated from the ionization constant of chlorous acid and the amount of hydrogen ion in solution produced by the partial ionization of the carboxylic acid. Thus the hydrogen ion concentration, [H⁺], in a solution of the carboxylic acid, HA, of known molar concentration and whose ionization constant is K_{A}, may be calculated from the following relationship:${\text{K}}_{\text{A}} \text{=} \frac{{\text{[H}}^{\text{+}} {\text{] [A}}^{\text{-}} \text{]}}{\text{[HA]}}$ This same relationship may be applied to calculate the relative chlorite and chlorous acid concentrations where the ionization constant for chlorous acid is 1.1 x 10⁻². That is:${\text{1.1 x 10}}^{\text{-2}} \text{=} \frac{{\text{[H}}^{\text{+}} {\text{] [ClO}}_{\text{2}} {\text{}}^{\text{-}} \text{]}}{{\text{[HClO}}_{\text{2}} \text{]}}$ where the hydrogen ion concentration, [H⁺], is the quantity already determined by ionization of the known amount of the carboxylic acid, HA. This calculation is well-known to those skilled in this art.

The oral hygiene compositions of this invention provide a metastable chlorous acid composition formed from relatively small amounts of chlorite and acid. This composition is capable of generating chlorine dioxide over a long period of time at continuing levels of effectiveness. As chlorine dioxide forms, more of the chlorite converts to chlorous acid by interacting with hydrogen ions further generated by ionization of the carboxylic acid.

The oral hygiene compositions of this invention are therefore different from many prior art chlorine dioxide generating compositions which consist of relatively high concentrations of chlorite and acid. Prior art chlorine dioxide-containing compositions were not used as mouthwashes or toothpastes and result in the rapid conversion of chlorous acid to chlorine dioxide. The rate of chlorine dioxide formation depends on the sum of the square of chlorous acid concentration and the product of chlorous acid and chlorite concentrations according to the equation:$\frac{{\text{d[ClO}}_{\text{2}} \text{]}}{\text{dt}} {\text{= K}}_{\text{1}} {\text{[HClO}}_{\text{2}} {\text{]}}^{\text{2}} {\text{+ [HClO}}_{\text{2}} {\text{] [ClO}}_{\text{2}} \text{]}$ See Gordon, "The Chemistry of Chlorine Dioxide," Prog. Inorg. Chem. 15:201 (1972).

Thus the rate at which chlorine dioxide forms depends exponentially on the amount of chlorite ion which is converted to chlorous acid and the amount of chlorite ion present.

In certain embodiments of the invention, the chlorous acid generating composition comprises an aqueous solution containing generally from about 0.01 to about 1, typically from about 0.02 to about 0.5, and preferably from about 0.03 to about 0.3 percent by weight of metal chlorite and a suitable amount of an organic acid having a pK of from about 2.8 to about 4.2. The pH of this composition is generally less than about 7, typically from about 2.2 to about 7.0.

In yet another embodiment of this invention, even lower concentrations of chlorite and acid may be used in the composition. These compositions comprise an aqueous solution containing generally up to about 0.4, and typically from about 0.0001 to about 0.03 percent by weight of metal chlorite, and a suitable amount of acid having a pK of from about 2.8 to about 4.2. The pH of the composition is generally less than about 7, typically from about 2.2 to about 7.0.

In certain embodiments of the invention, the carboxylic acid is an alpha-hydroxy carboxylic acid. In preferred embodiments, the alpha-hydroxy carboxylic acid has the structure of Formula I herein.

Optionally the compositions of the invention may contain either a suitable amount of a compound containing vicinal hydroxy groups or an amount of a water-soluble chloride in a significant molar excess to the chlorite, or both. Those optional ingredients may facilitate the formation of chlorine dioxide from chlorous acid and are thus useful in rapidly disinfecting compositions where an increased rate of chlorine dioxide formation is desired while maintaining a low concentration of chlorite and acid.

The metal chlorite useful in the present composition may more generally be described as a chlorine dioxide liberating compound. By "chlorine dioxide liberating compound" is meant any compound which, when appropriately treated, effects the production of chlorine dioxide as a result of a change in the valence state of the chlorine atom from +3 to +4. While any chlorine dioxide liberating compound may be used, water-soluble chlorites are preferred because they are readily available and inexpensive. Typical water-soluble chlorites include metal chlorites, such as alkali metal chlorites and alkaline earth metal chlorites. Sodium chlorite and potassium chlorite are preferred. Sodium chlorite is particularly preferred.

The flavoring agents useful in the compositions of this invention include any flavoring agent or mixture of flavoring agents acceptable in oral hygiene compositions. Such flavoring agents are well-known to those skilled in this art and include 1-carvone (mint flavor), peppermint oil, aspartame, saccharin, wintergreen oil, cinnamon oil, clove oil, menthol, thymol, eucalyptol, oil of sassafras, oil of anise, dextrose and levulose, and other flavoring agents well-known to those skilled in this art.

The term "oral hygiene compositions" is meant to include any composition which is used in the mouth in order to promote oral hygiene. These compositions may be in the form of a mouthwash, toothpaste, chewing gum, lozenge, or the like.

These compositions may be in the form of aqueous solutions, as in a mouthwash composition, gels, as in toothpaste or dentrifice compositions, solids, as in lozenges, or combined with fillers, as in chewing gum composition.

The compositions of this invention may contain other additives such as chelating agents (e.g., Na₄EDTA), or preservatives (e.g., sodium benzoate). The identity and amount of the other additives will depend upon the type of oral hygiene composition and its end use. Such additives are well-known to those skilled in the art. Suitable penetrants, astringents, deodorants, and other therapeutic or preventive compounds may be added. Dentrifices or toothpaste compositions according to the invention may also contain humectants, binders sudsing agents, abrasive polishing materials, and thickening agents.

The amount of chlorine dioxide liberating compound that may be used in this composition may be generally from about 0.01% to about 1%, typically from about 0.02% to about 0.05%, and preferably from about 0.03% to about 0.3% by height of the total composition (including the application medium).

At chlorite ion levels higher than about 0.5%, the concentration of chlorous acid formed upon admixture of a carboxylic acid may be in excess of that required for the formation of a metastable chlorous acid solution. These higher concentrations of chlorous acid would cause the formation of chlorine dioxide, through the degradation of chlorous acid at too rapid a rate.

Any acid of low toxicity may be used in the compositions of the invention so long as the chlorite ion concentration limits described above and the degree of conversion to chlorous acid are met. Preferably carboxylic acids are used. Preferred carboxylic acids include citric, malic, tartaric, glycolic, mandelic, salicylic and carbonic or other structurally similar acids as described in Formula I hereinbelow: 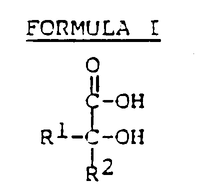
R¹ and R² may be the same or different and may be selected from the group consisting of hydrogen, methyl, -CH₂COOH, -CH₂OH, -CHOHCOOH, and -CH₂C_{6H5}.

The pK of these carboxylic acids may be generally from about 2.8 to about 4.2, and preferably from about 3.0 to about 4.0.

The amount of carboxylic acid used in these compositions should be sufficient to lower the pH of the composition to less than about 7, typically from about 2.5 to about 6, and preferably from about 3.0 to about 5.0. Furthermore, this amount may be generally from about 0.01% to about 3%, typically from about 0.5% to about 2%, and preferably from about 0.1% to about 1% by weight of the total composition (including the application medium).

The amount of flavoring agent useful in this invention may vary widely but is generally from about 0.01 to about 5, typically from about 0.02 to about 2, and preferably from about 0.05 to about 1 percent by weight based upon the total weight of the composition.

A suitable amount of a vicinal dihydroxy or polyhydroxy compound may also be added to the compositions of the present invention. The use of such compositions enables one to produce compositions according to the invention which are more rapidly effective in higher pH ranges. The use of these vicinal dihydroxy or polyhydroxy compounds also allows for the use of compositions according to the invention which contain a much lower acid concentration than that which is needed if the vicinal polyhydroxy compound comprising at least two vicinal hydroxy groups is absent.

Vicinal polyhydroxy compounds which contain at least two vicinal hydroxy groups are well-known to those skilled in this art and include dextrose and other sugars, glycerin, sorbitol, and inositols. In other embodiments, sugars with vicinal hydroxy groups in the cis configuration such as galactose, mannose, and ribose may be used.

The use of such vicinal polyhydroxy compounds, particularly those with cis-vicinal hydroxy groups, in conjunction with the chlorine dioxide liberating compound and carboxylic acid results in a synergistic composition. The vicinal dihydroxy or polyhydroxy compound catalyzes the formation of chlorine dioxide from chlorous acid. For example, the rate of formation of the active chlorine dioxide entity using a composition comprising sodium chlorite and mandelic acid is substantially enhanced by the addition of a relatively insubstantial amount of a vicinal polyhydroxy compound. Thus, the use of as little as 0.1% ribose in the composition substantially enhances the rate of formation of the active entity vis-a-vis a composition containing only sodium chlorite and one of the organic acids discussed hereinabove.

Stated otherwise, a composition containing the vicinal polyhydroxy compound may be prepared having substantially the same initial germ-killing efficacy in a specified time period as a composition which does not contain the vicinal polyhydroxy compound even though the composition containing the polyhydroxy compound contains substantially much less organic acid and sodium chlorite. However, such activation of the system results in a more rapid depletion of the chlorite ion in the composition, so that the germ-killing activity at a later time period would be less. The vicinal polyhydroxy compounds may also serve another purpose in the composition in that it may act as a sweetener or enhance the solubility of the flavoring agents.

The amount of vicinal polyhydroxy compound containing at least two vicinal hydroxy groups may vary widely, but in the present invention there is employed generally less than about 20%, typically from about 0.1% to about 10%, and preferably from about 0.2% to about 5% by weight of the total composition.

Alternatively, or in addition, the composition may contain a large excess of chloride ion in the form of an alkali or an alkaline earth metal salt. The excess may be from about a 10 to about a 100-fold excess by weight of chloride ion of total chlorite ion concentration. Large excesses of chloride ion in acid solutions (below a pH of about 7) cause the chlorite ion to decompose in an accelerated manner, via the formation of chlorous acid to form chlorine dioxide. In a preferred embodiment of the invention where rapid disinfection is required, the composition contains both a high excess of chloride ion and a sufficient amount of vicinal polyhydroxy compound comprising at least two vicinal hydroxy groups.

The chlorine dioxide liberating compound is generally kept separate from the organic acid prior to use in order to avoid premature reaction of the ingredients. The flavoring agent may be combined with either the organic acid or the chlorine dioxide liberating compound, or both, prior to their admixture.

The oral hygiene compositions of this invention result in improved bactericidal, fungicidal, virucidal and taste properties over presently available commercial oral hygiene compositions and aid in denial plaque reduction.

The present invention also provides a process for producing disinfectant oral hygiene compositions. This process comprises admixing: (a) a flavoring agent acceptable for oral hygiene compositions. (b) from about 0.03% to about 0.3% by weight of chlorine dioxide liberating compound based upon the total weight of the composition, and (c) a suitable amount of an organic acid which as a pK of from about 2.8 to about 4.2. The composition contains substantially no lactic acid.

By treating the mouth with an oral hygiene composition dental plaque can be reduced and gingivitis and periodonititis in the mouth can be controlled. The composition comprises (a) a flavoring agent acceptable for use in the mouth, (b) from about 0.03 to about 0.3 of a chlorine dioxide liberating compound based upon the total weight of the composition, and (c) a suitable amount of an organic acid having a pK of from about 2.8 to about 4.2. The composition contains substantially no lactic acid.

The present invention is illustrated by the following examples. Unless otherwise noted, all parts and percentages in the examples as well as the specification and claims are by weight.

### EXAMPLE I

This example illustrates the preparation of a toothpaste composition according to the present invention.

There is prepared a two-part disinfectant toothpaste composition according to the invention having a first base paste or gel and a second activator paste or gel.

The formulations of the two toothpaste parts on a percent weight to weight basis are as follows:

### TOOTHPASTE

| BASE | % W/W |
|---|---|
| Poly(sulfonic Acid) [16% aqueous solids] | 45.0 |
| Sodium hydroxide, 1N | 40.0 |
| Bentonite | 2.0 |
| Sodium lauryl sulfate | 1.0 |
| Titanium dioxide | 1.0 |
| Silica, amorphous | 0.5 |
| Sodium chlorite | 1.0 |
| Water | q.s |

| ACTIVATOR | |
|---|---|
| Glycerin | 10.0 |
| Magnesium aluminum silicate | 5.0 |
| Hydroxyethylcellulose | 2.0 |
| Malic acid | 1.5 |
| Flavor, wintergreen | 0.4 |
| FD&C Blue #1 (0.5% solution) | 0.06 |
| Sodium benzoate | 0.05 |
| Sodium saccharine | 0.05 |
| Water | q.s |

The pH of the composition resulting from the mixture of substantially equal portions of the base and activator gels of the above formulation is about 4.15.

The base gel and the activator gel are preferably stored separately prior to use, e.g., in a double-compartment tube. The two gels are mixed, preferably just prior to use, in substantially equal amounts and the mixture is used in the normal manner as a toothpaste. Alternatively, substantially equal portions of the gels are placed in the mouth and mixed by the brushing action while the subject brushes his or her teeth.

### EXAMPLE II

This example illustrates the properties of compositions employing hydroxyethyl cellulose as a gelling agent for sodium chlorite in the preparation of a final composition comprising sodium chlorite and lactic acid.

Example I is repeated, but hydroxyethyl cellulose is used at the 2% level in place of the polysulfonic acid salt and excluding the sodium hydroxide. This gel is not storage stable for a commercially acceptable period of time. The cellulose gelling agent depolymerizes and loses viscosity.

### EXAMPLE III

This example illustrates the preparation of a mouthwash according to the present invention.

A first solution is prepared by dissolving 0.4 grams of technical grade sodium chlorite, 0.17 grams of powdered Na₄EDTA 4H₂O, 0.5 grams of 1-carvone (mint flavor), and the appropriate amount of a compatible food grade yellow dye in 500 milliliters of aqueous solution. A second solution is prepared by dissolving 1.375 grams of anhydrous citric acid and the appropriate amount of FD&C Blue #1 in a batch of 500 milliliters of a 10 percent by weight aqueous solution of glycerin.

The two solutions are mixed, preferably just prior to use, in substantially equal amounts and the mixture is used in the normal manner as a mouthwash.

### EXAMPLE IV

This example also illustrates the preparation of a mouthwash according to the present invention.

A first aqueous solution of 0.25% sodium chlorite and 0.10% NaOH in deionized water is prepared.

A second aqueous activator solution is prepared of 0.75% malic acid, 10.00% glycerine USP, 0.05% sodium benzoate, 0.50% Equal (aspartame diluted with dextrose and corn syrup solids), 0.03% FD&C Blue #1 (0.3% solution), and 0.40% Wintergreen Flavor (BBA), in deionized water.

The two solutions are mixed, preferably just prior to use in substantially equal amounts, and the mixture is used in the normal manner as a mouthwash.

### EXAMPLE V

This example illustrates the ability of a composition of the present invention to reduce dental plaque and control gingivitis and periodontitis.

Mouthwash compositions according to the invention were used in a five day triple-crossover plaque reduction test. In this test, 18 human subjects were divided into three groups of six subjects each. The teeth of each subject were scraped free of plaque immediately prior to the commencement of each five day period.

Each group was subjected to three five day periods of treatment with the following formulations: (a) placebo, (b) low potency mouthwash formulation, and (c) high potency mouthwash formulation. The sequence of the treatments per group were selected in a random fashion. During the five day period the subjects rinsed with the formulation twice daily, i.e., in the morning and afternoon. The subjects did not brush their teeth or perform any other oral hygiene procedures. The same treatment procedure was followed for all three of the formulations. Plaque scores after each five day period were determined by a modified Quigley-Hein index.

The placebo was formulated so that it had substantially the same perceived acidity as the other two formulations. The placebo formulation comprised an aqueous solution of 0.27% citric acid, 0.08% sodium chloride, 0.5% 1-carvone, 0.09% FD&C Green #3 (5000 ppm solution), 0.03% FD&C Yellow #5 (5000 ppm solution), and 5.0% glycerin.

The compositions were formulated in two parts, A and B. Equal volumes of the two parts were mixed immediately before rinsing. The percent compositions of parts A and B of the high potency and low potency formulations were as follows:

| INGREDIENT | HIGH POTENCY | LOW POTENCY |
|---|---|---|
| Part A | | |
| Sodium chlorite (79%) | 0.32 | 0.08 |
| 1-Carvone | 0.10 | 0.10 |
| FD&C Green #3 (5000 ppm solution) | 0.18 | 0.18 |
| 1 N Sodium hydroxide | q.s. | q.s. |

| Part B | | |
|---|---|---|
| Malic acid | 0.75 | 0.275 |
| Sodium benzoate | 0.05 | 0.05 |
| Glycerin | 10.00 | 10.00 |
| FD&C Yellow #5 (5000 ppm solution) | q.s. | q.s. |

The average plaque scores of the subjects listed below show that the mouthwash compositions of this invention were effective in substantially reducing dental plaque.

| | Score (average) | % Reduction |
|---|---|---|
| Placebo | 3.82 | --- |
| Low potency | 3.35 | 12.3 |
| High potency | 2.47 | 35.3 |

### EXAMPLE VI

An evaluation was made of the microbiocidal effectiveness of the High Potency mouthwash (0.32% Sodium chlorite - Part A; 0.75% Malic acid - Part B) in Example V, versus that of 0.2% chlorhexidine digluconate. The 0.2% Chlorhexidine digluconate solution is the recommended reference standard by which other oral hygiene germicides should be measured. The procedure employed was based on the method in the FDA OTC Oral Health Care Monograph (Federal Register, Vol. 47, No. 101, May 25, 1982, pages 22890-22900). The test organisms were:
Candida albicans ATCC 18804 (American Type Culture Collection, Rockville, Md.)
Actinobacillus actinomycetemcomitans Forsyth Clinic Isolate #Y4
Streptococcus mutans ATCC 25175
The microorganisms were grown in Brain Heart Infusion, and subcultured for two successive days at 37°C. Two ml of a 50:50 mix of each test organism in sterile fetal calf serum was added to 8 ml of the mouthwash formulation, and triplicate aliquots of the inoculated test formulation were removed at 30 and 60 second for subculture. C. albicans was grown and subcultured aerobically; all other organisms, anaerobically. The High Potency test solutions were neutralized first in fluid thioglycolate medium, and further neutralized in the plate count agar. The chlorhexidine digluconate control solutions were only neutralized in the enumeration agar. Results obtained were as follows:

| Formulation | Test Organism | Average Microbial Log Reduction* | |
|---|---|---|---|
| | | 30 Sec. Exp. | 60 Sec. Exp. |
| High Potency | C. albicans | 3.2 | 3.0 |
| | Actinobacillus | 7.5 | 7.5 |
| | Strep. mutans | 3.5 | 4.3 |
| Chlorhexidine digluconate, 0.2% | C. albicans | 3.2 | 3.3 |
| | Actinobacillus | 6.6 | 6.3 |
| | Strep. mutans | 3.0 | 3.0 |

| | | | |
|---|---|---|---|
| *Average of three results. | | | |

The High Potency Formulation shows a microbiocidal efficacy equal to or greater than of chlorhexidine digluconate at the 0.2% level.

These microbiocidal data strongly indicate that these formulations will not only be helpful in reducing dental plaque formation, but also would inhibit the onset and severity of gingivitis and other associated oral disorders such as periodontitis which could lead to eventual tooth loss.

## Claims

1. A composition for disinfecting a substrate comprising two parts, wherein said first part comprises an aqueous solution or gel containing a suitable amount of a protic acid; and wherein said second part comprises an aqueous solution or gel containing a metal chlorite such that, when the parts are combined, the chlorite ion concentration in the form of chlorous acid is not more than 15 % by weight of the total amount of chlorite ion concentration, with the proviso that said composition contains substantially no lactic acid.

2. The composition of claim 1 wherein said composition contains from 0.01 % to 0.5 % by weight metal chlorite based upon the total weight of said composition.

3. The composition of claim 1 wherein said compositions contains from 0.03 % to 0.3 % by weight metal chlorite based upon the total weight of said composition.

4. The composition of claim 1 wherein said composition contains from 0.02 % to 0.05 % by weight metal chlorite based upon the total weight of said composition.

5. The composition of claim 1 wherein said metal chlorite is sodium chlorite.

6. The composition of claim 1 wherein said composition contains from 0.01 % to 3 % by weight protic acid based upon the total weight of said composition.

7. The composition of claim 1 wherein said composition contains from 0.5 % to 2 % by weight protic acid based upon the total weight of said composition.

8. The composition of claim 1 wherein said composition contains from 0.1 % to 1 % by weight protic acid based upon the total weight of said composition.

9. The composition of claim 1 wherein protic acid is an organic acid having a pK from 2.8 to 4.2, wherein the pH of said composition is from 2.2 to 7.0.

10. The composition of claim 9 wherein said organic acid is selected from the group consisting of citric, malic, tartaric, glycolic, mandelic, salicylic and carbonic acids.

11. The composition of claim 1 further comprising a flavoring agent.

12. The composition of claim 11 wherein said flavoring agent is selected from the group consisting of 1-carvone, aspartame, saccharin, peppermint oil, wintergreen oil, cinnamon oil, clove oil, menthol, thymol or eucalyptol.

13. The composition of claim 1 further comprising at least a 10-fold molar excess of a water-soluble chloride ion compared to the total concentration of chlorite ion.

14. The composition of claim 1 further comprising a compound that contains at least two vicinal hydroxy group.

15. A process for producing a disinfecting composition comprising admixing two parts, wherein said first part comprises an aqueous solution or gel containing a suitable amount of a protic acid and wherein said second part comprises an aqueous solution or gel containing a metal chlorite such that, when the parts are combined, the chlorite ion concentration in the form of chlorous acid is not more than 15 % by weight of the total amount of chlorite ion concentration, with the proviso that said composition contains substantially no lactic acid.

16. The process of claim 15 wherein at least one of said first and second parts comprises a flavoring agent.

17. The process of claim 15 wherein said protic acid is an organic acid having a pK of from 2.8 to 4.2, and wherein the metal chlorite is present in said second part in an amount such that said composition contains from 0.03 % to 0.3 % by weight of metal chlorite based upon the total weight of said composition.

## Patentansprüche

1. Zusammensetzung zum Desinfizieren eines Substrats, umfassend zwei Teile, worin der erste Teil eine wäßrige Lösung oder ein Gel, enthaltend eine geeignete Menge einer Protonensäure, umfaßt; und worin der zweite Teil eine wäßrige Lösung oder ein Gel, enthaltend ein Metallchlorit, umfaßt, so daß, wenn die Teile kombiniert werden, die Chloritionenkonzentration in Form von chloriger Säure nicht mehr als 15 Gew.-% der Gesamtmenge der Chloritionenkonzentration beträgt, mit der Maßgabe, daß die Zusammensetzung im wesentlichen keine Milchsäure enthält.

2. Zusammensetzung nach Anspruch 1, worin die Zusammensetzung 0,01 bis 0,5 Gew.-% Metallchlorit, bezogen auf das Gesamtgewicht der Zusammensetzung, enthält.

3. Zusammensetzung nach Anspruch 1, worin die Zusammensetzung 0,03 bis 0,3 Gew.-% Metallchlorit, bezogen auf das Gesamtgewicht der Zusammensetzung, enthält.

4. Zusammensetzung nach Anspruch 1, worin die Zusammensetzung 0,02 bis 0,05 Gew.-% Metallchlorit, bezogen auf das Gesamtgewicht der Zusammensetzung, enthält.

5. Zusammensetzung nach Anspruch 1, worin das Metallchlorit Natriumchlorit ist.

6. Zusammensetzung nach Anspruch 1, worin die Zusammensetzung 0,01 bis 3 Gew.-% Protonensäure, bezogen auf das Gesamtgewicht der Zusammensetzung, enthält.

7. Zusammensetzung nach Anspruch 1, worin die Zusammensetzung 0,5 bis 2 Gew.-% Protonensäure, bezogen auf das Gesamtgewicht der Zusammensetzung, enthält.

8. Zusammensetzung nach Anspruch 1, worin die Zusammensetzung 0,1 bis 1 Gew.-% Protonensäure, bezogen auf das Gesamtgewicht der Zusammensetzung, enthält.

9. Zusammensetzung nach Anspruch 1, worin die Protonensäure eine organische Säure mit einem pK von 2,8 bis 4,2 ist, worin der pH der Zusammensetzung 2,2 bis 7,0 beträgt.

10. Zusammensetzung nach Anspruch 9, worin die organische Säure gewählt ist aus der Gruppe bestehend aus Citronensäure, Äpfelsäure, Weinsäure, Glycolsäure, Mandelsäure, Salicylsäure und Kohlensäure.

11. Zusammensetzung nach Anspruch 1, welche weiterhin einen Geschmacksstoff umfaßt.

12. Zusammensetzung nach Anspruch 11, worin der Geschmacksstoff gewählt ist aus der Gruppe bestehend aus l-Carvon, Aspartame, Saccharin, Pfefferminzöl, Wintergrünöl, Zimtöl, Nelkenöl, Menthol, Thymol oder Eukalyptol.

13. Zusammensetzung nach Anspruch 1, weiterhin umfassend mindestens einen 10-fachen molaren Überschuß eines wasserlöslichen Chloridions, bezogen auf die Gesamtkonzentration des Chloritions.

14. Zusammensetzung nach Anspruch 1, weiterhin umfassend eine Verbindung, die mindestens zwei vicinale Hydroxygruppen enthält.

15. Verfahren zum Herstellen einer desinfizierenden Zusammensetzung, umfassend das Vermischen von zwei Teilen, worin der erste Teil eine wäßrige Lösung oder ein Gel, enthaltend eine geeignete Menge einer Protonensäure, umfaßt und worin der zweite Teil eine wäßrige Lösung oder ein Gel, enthaltend ein Metallchlorit, umfaßt, so daß, wenn die Teile kombiniert werden, die Chloritionenkonzentration in Form von chloriger Säure nicht mehr als 15 Gew.-% der Gesamtmenge der Chloritionenkonzentration beträgt, mit der Maßgabe, daß die Zusammensetzung im wesentlichen keine Milchsäure enthält.

16. Verfahren nach Anspruch 15, worin mindestens einer von dem ersten und zweiten Teil einen Geschmacksstoff umfaßt.

17. Verfahren nach Anspruch 15, worin die Protonensäure eine organische Säure mit einem pK von 2,8 bis 4,2 ist, und worin das Metallchlorit in dem zweiten Teil in einer solchen Menge vorhanden ist, daß die Zusammensetzung 0,03 bis 0,3 Gew.-% Metallchlorit, bezogen auf das Gesamtgewicht der Zusammensetzung, enthält.

## Revendications

1. Composition de désinfection d'un substrat comprenant deux parties, dans laquelle la première partie comprend une solution aqueuse ou un gel contenant une quantité convenable d'acide protonique ; et dans laquelle la seconde partie comprend une solution aqueuse ou un gel contenant un sel chloreux métallique de sorte que, quand les parties sont combinées, la concentration en ions chlorites sous foie d'acide chloreux n'est pas supérieure à 15 % en poids de la concentration totale en ions chlorites, sous réserve que la composition ne contienne pratiquement pas d'acide lactique.

2. Composition selon la revendication 1, dans laquelle la composition contient de 0,01 % à 0,5 % en poids de sel chloreux métallique par rapport au poids total de la composition.

3. Composition selon la revendication 1, dans laquelle la composition contient de 0,03 % à 0,3 % en poids de sel chloreux métallique par rapport au poids total de la composition.

4. Composition selon la revendication 1, dans laquelle la composition contient de 0,02 % à 0,05 % en poids de sel chloreux métallique par rapport au poids total de la composition.

5. Composition selon la revendication 1, dans laquelle le sel chloreux métallique est un chlorite de sodium.

6. Composition selon la revendication 1, dans laquelle la composition comprend de 0,01 % à 3 % en poids d'acide protonique par rapport au poids total de la composition.

7. Composition selon la revendication 1, dans laquelle la composition comprend de 0,5 % à 2 % en poids d'acide protonique par rapport au poids total de la composition.

8. Composition selon la revendication 1, dans laquelle la composition comprend de 0,1 % à 1 % en poids d'acide protonique par rapport au poids total de la composition.

9. Composition selon la revendication 1, dans laquelle l'acide protonique est un acide organique ayant un pK de 2,8 à 4,2, le pH de la composition étant de 2,2 à 7,0.

10. Composition selon la revendication 9, dans laquelle l'acide organique est choisi dans le groupe comprenant les acides citrique, malique, tartrique, glycolique, mandélique, salicylique et carbonique.

11. Composition selon la revendication 1, comprenant en outre un agent de saveur.

12. Composition selon la revendication 11, dans laquelle l'agent de saveur est choisi dans le groupe comprenant la 1-carvone, l'aspartame, la saccharine, l'essence de menthe poivrée, l'essence de wintergreen, l'essence de cannelle, l'essence de clous de girofle, le menthol, le thymol ou l'eucalyptol.

13. Composition selon la revendication 1, comprenant en outre au moins un excès molaire de 10 fois d'ions chlorures solubles dans l'eau par rapport à la concentration totale d'ions chlorites.

14. Composition selon la revendication 1, comprenant en outre un composé qui contient au moins deux groupes hydroxy voisins.

15. Procédé d'obtention d'une composition désinfectante consistant à mélanger deux parties, dans lequel la première partie comprend une solution aqueuse ou un gel contenant une quantité convenable d'acide protonique et dans lequel la seconde partie comprend une solution aqueuse ou un gel contenant un sel chloreux métallique de sorte que, quand les parties sont combinées, la concentration en ions chlorites sous forme d'acide chloreux n'est pas supérieure à 15 % en poids de la concentration totale en ions chlorites sous réserve que la composition ne contienne sensiblement pas d'acide lactique.

16. Procédé selon la revendication 15, dans lequel au moins une des première et seconde parties comprend un agent de saveur.

17. Procédé selon la revendication 15, dans lequel l'acide protonique est un acide organique ayant un pK de 2,8 à 4,2 et dans lequel le sel chloreux métallique est présent dans la seconde partie en une quantité telle que la composition contienne de 0,03 % à 0,3 % en poids de sel chloreux métallique par rapport au poids total de la composition.
